# EUROPEAN PATENT APPLICATION

(11) **EP 4 480 421 A1**
(43) Date of publication of application: **25.12.2024**
(21) Application number: 23181236.3
(22) Date of filing: 23.06.2023
(51) Int. Cl.: A61B 6/10, A61B 6/00

(54) **RADIATION INFORMATION FOR A CURRENT SITUATION**

(71) Applicant: Koninklijke Philips N.V., 5656 AG Eindhoven (NL)
(72) Inventor: BACHVAROV, Chavdar, Eindhoven (NL); VAN RIEL, Sjors, Eindhoven (NL); BOON, Sjirk, 5656AG Eindhoven (NL); HENDRIKS, Bernardus Hendrikus Wilhelmus, Eindhoven (NL); KESSELS, Angelique Carin Johanna Maria, Eindhoven (NL); NETO FONSECA, Lucia Teresa, Eindhoven (NL)
(74) Representative: Philips Intellectual Property & Standards

(57) **Abstract**

The present invention relates to radiation protection. In order to provide improved radiation protection measures with a facilitated handling, a device (10) for providing radiation information for a current situation is provided that comprises a data input (12), a data processor (14) and an output interface (16). The data input is configured: to provide image data (18) about a current scene in a medical room, the image data representing at least one staff member and equipment in the medical room; to provide information (20) about a spatial arrangement of an X-ray imaging device within the medical room; and to provide imaging parameters (22) of a planned X-ray imaging procedure. The data processor is configured: to detect objects in the image data and to determine their position within the medical room; to detect the at least one staff member in the image data and to determine, in the image data, a parametrized pose of the detected at least one staff member within the medical room; to calculate a prediction of X-ray radiation within the medical room that would occur during the planned X-ray imaging procedure, based on: the determined positions of the detected objects and the information about the spatial arrangement and imaging parameters; to determine an amount of radiation received by at least two different body parts of the at least one staff member, based on the calculated prediction and the determined pose of the detected at least one staff member; and to generate radiation information for the at least one staff member. The output interface is configured to provide the radiation information to the at least one staff member based on the determined amount of radiation.

## Description

### FIELD OF THE INVENTION

The present invention relates to radiation protection in medical rooms in general, and relates in particular to a device for providing radiation information for a current situation, to a system for providing radiation information for a current X-ray imaging procedure, to an X-ray imaging arrangement with radiation information prediction and to a method for providing radiation information for a current situation.

### BACKGROUND OF THE INVENTION

With the rise of minimal invasive procedures and concerns over staff shortage and turnover, radiation safety and hygiene continue to be an important metric for safety and well-being of both patients and staff. Although radiation is invisible to the human eye, its health effects can be serious. Measures are provided to make the staff more aware and safer during procedures. As an example, dose badges are provided that are worn by the user. As a further example, US 10172575 B2 describes a protection system for protecting a person against X-ray scatter radiation. However, it has been shown that radiation dose may vary throughout the room and radiation protection may be cumbersome to take care off.

### SUMMARY OF THE INVENTION

There may thus be a need to provide improved radiation protection measures with a facilitated handling.

The object of the present invention is solved by the subject-matter of the independent claims; further embodiments are incorporated in the dependent claims. It should be noted that the following described aspects of the invention apply also for the device for providing radiation information for a current situation, for the system for providing radiation information for a current X-ray imaging procedure, for the X-ray imaging arrangement with radiation information prediction and for the method for providing radiation information for a current situation.

According to the present invention, a device for providing radiation information for a current situation is provided. The device comprises a data input, a data processor and an output interface. The data input is configured to provide image data about a current scene in a medical room, the image data representing at least one staff member and equipment in the medical room. The data input is also configured to provide information about a spatial arrangement of an X-ray imaging device within the medical room. The data input is further configured to provide imaging parameters of a planned X-ray imaging procedure. The data processor is configured to detect objects in the image data and to determine their position within the medical room. The data processor is also configured to detect the at least one staff member in the image data and to determine, in the image data, a parametrized pose of the detected at least one staff member within the medical room. The data processor is further configured to calculate a prediction of X-ray radiation within the medical room that would occur during the planned X-ray imaging procedure, based on: the determined positions of the detected objects and the information about the spatial arrangement and imaging parameters. The data processor is also configured to determine an amount of radiation received by at least two different body parts of the at least one staff member, based on the calculated prediction and the determined pose of the detected at least one staff member. The data processor is configured to generate radiation information for the at least one staff member. The output interface is configured to provide the radiation information to the at least one staff member based on the determined amount of radiation.

As a result, the staff members are provided with more accurate information about an upcoming radiation dose. The staff members are stimulated to take action in order to reduce their received dosed during X-ray imaging. As a result, radiation protection is achieved.

As an effect, radiation exposure is determined for the complete body, thus avoiding missing locations like the radiation exposure of the eyes and other important organs of the body.

This allows a prediction of radiation in a facilitated manner in order to increase awareness, and even trigger improvement action by the staff members.

According to an example, the parametrized pose of the detected at least one staff member within the medical room is based on a stick-figure model.

Such simplified model is a stable and reliable approach to grasp the current situation of the user. The approach allows to rely on current or live image data and avoids pose trackers or other equipment.

According to an example, the radiation information comprises a visualization of the radiation distribution in the spatial context in relation to the at least one staff member and the staff member's at least two body parts.

The radiation information is thus provided in an intuitive way supporting the goal to make the user adapting pose or workflow in order to minimize radiation impact, i.e. dose.

According to an example, the data processor is configured to determine the amount of radiation as a prediction before an actual imaging takes place.

Thus, measures can be taken beforehand and not during radiation.

According to an example, for providing the information about the spatial arrangement of the X-ray imaging device within the medical room, the data processor is configured to detect the X-ray imaging device in the image data and to determine its spatial arrangement.

As an effect, the concept is based mainly on image data allowing an easy to implement and easy to operate setup.

According to an example, the data input is configured to provide information about radiation protective measures within the medical room. The data processor is configured to determine the amount of radiation also based on the information about the radiation protective measures.

This enables a more accurate information to the user, as the radiation protective measures can vary.

As an option, for providing the information about the radiation protective measures, the data processor is configured to analyze image data for identifying radiation protective measures and for determining their spatial arrangement.

Referring to image data provides a concept that needs other data, i.e. non-image data, only to a minimal extent.

According to an example, the image data is provided as data from an optical camera.

According to an example, the output interface is configured to present the radiation information as visualization on surface parts within the medical room.

Such presentation is intuitive and available for the whole team in order to also support each other when it comes to saving radiation impact on the staff members.

According to the present invention, also a system for providing radiation information for a current X-ray imaging procedure is provided. The system comprises a device for providing radiation information for a current situation according to one of the preceding examples and at least one camera. The at least one camera provides the image data about the current scene in the medical room.

According to the present invention, also an X-ray imaging arrangement with radiation information prediction is provided. The arrangement comprises a system for providing radiation information for a current X-ray imaging procedure according to the preceding example and an X-ray imaging system. The X-ray imaging system is configured to perform the planned X-ray imaging procedure. The X-ray imaging system provides the imaging parameters of the planned X-ray imaging procedure to the device for providing radiation information for the current situation.

According to the present invention, also a method for providing radiation information for a current situation is provided. The method comprises the following steps:
- Providing image data about a current scene in a medical room; the image data represents at least one staff member and equipment in the medical room.
- Detecting objects in the image data and determining their position within the medical room.
- Detecting the at least one staff member in the image data.
- Determining, in the image data, a parametrized pose of the detected at least one staff member within the medical room.
- Providing information about a spatial arrangement of an X-ray imaging device within the medical room.
- Providing imaging parameters of a planned X-ray imaging procedure.
- Calculating a prediction of X-ray radiation within the medical room that would occur during the planned X-ray imaging procedure, based on the determined positions of the detected objects, the information about the spatial arrangement and as the imaging parameters.
- Determining an amount of radiation received by at least two different body parts of the at least one staff member, based on the calculated prediction and the determined pose of the detected at least one staff member.
- Generating radiation information for the at least one staff member.
- Providing the radiation information to the at least one staff member based on the determined amount of radiation.

According to an aspect, a current scene is detected based on image data by detecting staff members in the images and by applying a simplified model to determine their pose. The possible radiation including scatter radiation is predicted and provided as radiation information to the user. This provides awareness e.g. before the actual radiation occurs. The user can therefore also adjust the pose, position or imaging scheme or other parameters to minimize dose impact. The prediction requires images from the current scene and some imaging parameters for the X-ray radiation procedure. To calculate radiation information for a current situation, only a minimal number of data sources and equipment is required.

According to an aspect, a pose adjustment advice is provided to reduce radiation harm. In one example, a camera-based AI (artificial intelligence) pose estimation algorithm is provided that refers to a so-called stick figure model of the user.

According to an aspect, the pose adjustment advice attempts to visualize the scatter radiation potential hazard to the physician before the actual X-ray investigation starts. In an option, this potential hazard is made visible at the level of body parts, such as head, chest, hands. The staff is thus made aware of the radiation hazard as function of their various body parts.

Briefly said, radiation safety hazards are made visible even when the X-ray system is not radiating.

The above approach can be used where X-ray systems are employed.

According to an aspect, the potential scatter radiation hazard as a function of the body parts of the personnel in the cathlab is made visible to the physician before the X-ray investigation starts in a reliable and in a computational cost-effective way. In particular, the staff is made aware of the radiation hazard as function of their various body parts.

According to an aspect, it is provided to calculate X-ray safe zones in an examination room using staff positions (using AI based 3D-pose detection personnel (stick figure), detecting lead apron) and shield positions in 3D space combined with IGT system settings, e.g. CWIS and/or Philips Open Innovation Platform) and to visualize it for the staff before they start radiation. The 3D positions are calculated by matching objects detected using AI algorithms on video from multiple calibrated cameras in the room. These are combined with a model of the scatter radiation that would be emitted based on the system C-arm and table positions as well as the X-ray settings. Furthermore, for each person the pose is calculated by using e.g. the stick figure model and/or the type of lead apron. From the pose, the radiation scatter model, and if applicable, the lead aprons, the expected radiation dose on each organ per person in the room is calculated and shown to the physician.

The goal is to calculate a 3D model of the scatter radiation shadow, created by protection shield(s), and the position of the body parts of the people (staff or patient) relative to that shadow.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for providing radiation information for a current situation.
Fig. 2 schematically shows an example of a system for providing radiation information for a current X-ray imaging procedure.
Fig. 3 shows an example of an X-ray imaging arrangement with radiation information prediction.
Fig. 4 shows basic steps of an example of a method for providing radiation information for a current situation.
Fig. 5 shows an example of a working scheme.
Fig. 6 shows another example of a working scheme.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

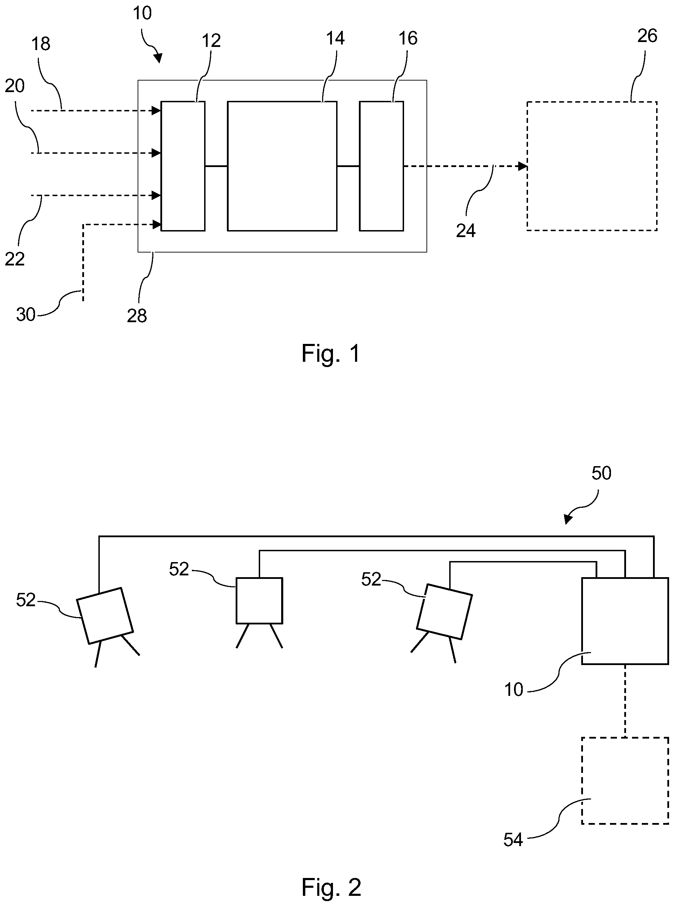

Fig. 1 schematically shows an example of a device 10 for providing radiation information for a current situation. The device 10 comprises a data input 12, a data processor and an output interface 16. The data input 12 is configured to provide image data 18 about a current scene in a medical room, the image data representing at least one staff member and equipment in the medical room. The data input 12 is also configured to provide information 20 about a spatial arrangement of an X-ray imaging device within the medical room. The data input 12 is further configured to provide imaging parameters 22 of a planned X-ray imaging procedure. The data processor 14 is configured to detect objects in the image data and to determine their position within the medical room. The data processor 14 is also configured to detect the at least one staff member in the image data and to determine, in the image data, a parametrized pose of the detected at least one staff member within the medical room. The data processor 14 is further configured to calculate a prediction of X-ray radiation within the medical room that would occur during the planned X-ray imaging procedure, based on: the determined positions of the detected objects and the information about the spatial arrangement and imaging parameters. The data processor 14 is furthermore configured to determine an amount of radiation received by at least two different body parts of the at least one staff member, based on the calculated prediction and the determined pose of the detected at least one staff member. The data processor 14 is also configured to generate radiation information for the at least one staff member. The output interface 16 is configured to provide (indicated with reference numeral 24) the radiation information to the at least one staff member based on the determined amount of radiation.

As an option, a display or presentation device 26 is provided, indicated with a dotted-line frame, in order to provide the radiation information to the at least one staff member.

A further frame 28 indicates that the data input 12, the data processor 14 and the output interface 16 are provided within a common housing as an option. In another option, the data input 12, the data processor 14 and the output interface 16 are provided separately.

The detection of the pose, also referred to as posture, of the personnel to determine the body parts like hands and head, e.g. the eyes, allows to determine radiation hazards per body part, for example for at least two body parts or portions. Detection of body parts like eyes and hands are of importance, since for instance the eyes may lead to cataract disease and the hands are often much closer to the radiation source during surgical procedures where the physician has to position a body part of the patient in the radiation beam.

The term "image data" relates to camera image depicting the current scene in such a way that the spatial arrangement of the different obstacles, equipment, devices, personnel etc. can be determined by the image.

The term "spatial arrangement" relates to the location, position and orientation of the device within the medical room.

The medical room is a space used for medical purposes like interventions, examinations and others. An example for a medical room is an operation room, an emergency care unit, an X-ray imaging room or a catheterization laboratory.

The term "imaging parameters" relates to settings used for imaging, such as viewing direction, viewing angle, location and orientation of the X-ray source, applied X-ray energy and so forth. In an option, the imaging parameters also comprise data relating to the subject to be imaged.

The term "subject" may also be referred to as individual. The "subject" may further also be referred to as patient, although it is noted that this term does not indicate whether any illness or disease is actually present with the subject.

The term "objects" relates to all different kinds of equipment used in the medical room. The objects can be movable or stationary. The objects comprise, among others, subject support, imaging equipment, monitoring equipment, lead shields, stools, separations, shelfs, trolleys, supports and displays.

The term "staff member" relates to the personnel present in the medical room. The staff member can be a surgeon, a physician monitoring the subject, a nurse, care taker or technical support.

The term "pose" relates to the posture of the staff member, such as standing upright or bending or leaning forward, or arms and hands towards the subject or reaching upwards.

The term "parametrized pose" relates to an approach trying to characterize the pose of the staff member in a simplified manner by referring to a reduced number of parameters that describe the pose of the staff member. By determining those parameters in the image data, the pose can be identified based on the parameters. The parametrized model reduces the staff member, with his/her corps and extremities like legs with feet, arms with hands and the head connected to each other to a simplified structure in which different parameters like lines are representing the different parts, connected in a pivoting manner, reflecting the different kind of joints like the hip joint, the knee or the elbow.

The term "prediction of X-ray radiation" relates to radiation that will most probably occur when performing the planned imaging procedure. The radiation comprises direct and scattered or stray radiation.

The term "radiation information" relates to information about an expected dose of radiation. The radiation information can be a map or color coding relating to the staff members body portions.

The radiation information relates to an expected exposure to X-ay radiation. The radiation information may also comprise advice for reaction, like changing a position or pose or even changing teg imaging settings or viewing directions.

In an option, the radiation prediction is based on the image data provided by e.g. cameras. A further data source are the imaging parameters supplied.

The term "data input" relates to providing or supplying data for data processing steps. The data input 12 can also be referred to as image data input. The data input 12 can also be referred to as data supply, as image data supply, as image input, as input unit or simply as input. In an example, the (image) data input 12 is data-connectable to an imaging source arrangement.

The term "data processor" relates to a processor or part of a processor arrangement that is provided to conduct the computing steps using the data supplied by the data input. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a display arrangement or display device. In another example, the output interface 16 is data-connected to a display. As an example, the signals by the controller can be provided by the output interface 16.

In an example, not shown in detail in Fig. 1, the parametrized pose of the detected at least one staff member within the medical room is based on a stick-figure model.

By using a stick figure model as an example of a parametrized pose model, a degree of simplification is achieved on the one side, but on the other hand, this simpler person model also allows much faster computation, e.g. in real time, as compared to, for example, using tracking individuals based on a voxel model.

The term "stick-figure model" relates to a model that reduces the staff member with his/her corps and extremities like legs with feet, arms with hands and the head to a plurality of sticks, or stick-like elements, connected to each other pivoting manner reflecting the different kind of joints like the hip joint, the knee or the elbow.

In an example, not shown in detail, the radiation information comprises a visualization of the radiation distribution in the spatial context in relation to the at least one staff member and the staff member's at least two body parts.

The term "visualization of the radiation distribution" relates to some sort of presentation easy to understand by the user. The visualization can be provided as a separate display or overlaid to an image of the scene or even overlaid to the staff members in situ.

In an example, not shown in detail, the data processor is configured to determine the amount of radiation as a prediction before an actual imaging takes place.

In an option, the visualization of the radiation distribution also takes into account a dose already applied to the staff member. For example, a dose tracking function is implemented.

In an example, not shown in detail, for providing the information about the spatial arrangement of the X-ray imaging device within the medical room, the data processor 14 is configured to detect the X-ray imaging device in the image data and to determine its spatial arrangement.

In an example, not shown in detail, the data input 12 is configured to provide information (indicated with a further dotted line arrow 30) about radiation protective measures within the medical room. The data processor 14 is configured to determine the amount of radiation also based on the information about the radiation protective measures. As an option, for providing the information about the radiation protective measures, the data processor 14 is configured to analyze image data for identifying radiation protective measures and for determining their spatial arrangement.

The protective measures relate to any measure applied to provide some sort of protection against radiation, such as shieldings, separations, walls, partitions and aprons.

In an example, not shown in detail, the output interface 16 is configured to provide a temporal presentation of the determined radiation in order to show where and when radiation is occurring.

In an example, a video sequence is provided for understanding changes in radiation dose occurring during a procedure. In an option, this is provided as a simulation for a planned procedure. In another option, this is provided as a video sequence of a procedure that has already taken place.

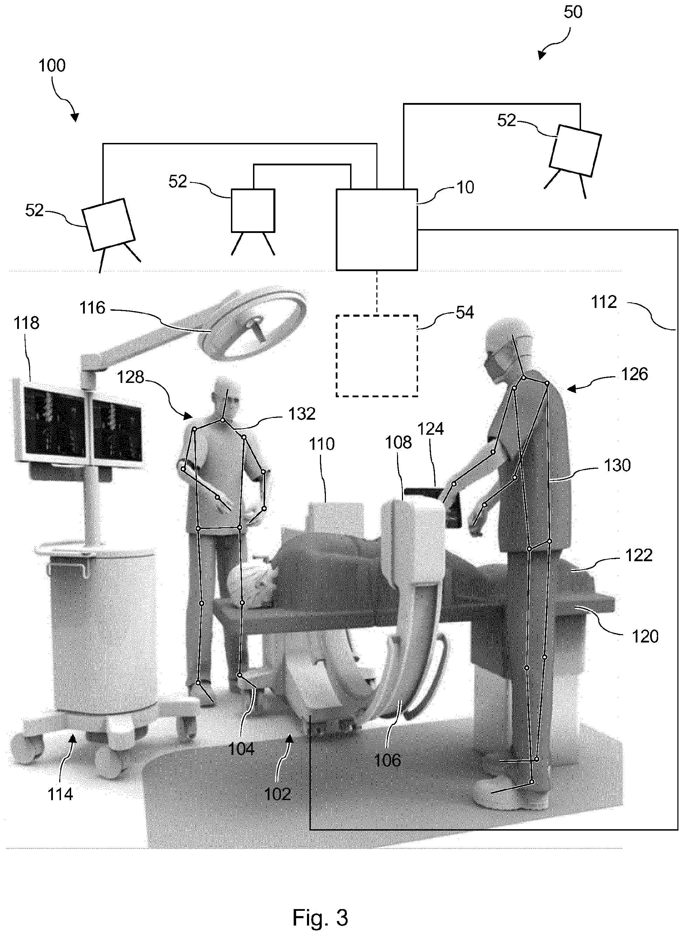

In an example, not shown in detail, the image data is provided as data from at least one optical camera (see also Fig. 2 and Fig. 3).

In an option, the image data is provided as data from depth cameras, e.g. from time of flight cameras.

In an example, the optical cameras operate based on visible light.

In an example, the image data represents at plurality of staff members. The data processor 14 is configured provide a parametrized pose like the stick-figure representation for a at least a part of the plurality of staff members.

In an example, not shown in detail, the information about the spatial arrangement of the X-ray imaging device within the medical room also comprises data of a subject support.

As an option, the image data also comprises image data of a subject.

In an example, not shown in detail, the data processor 14 is configured to update the determined amount of radiation constantly reflecting changes in the current scene.

This is mainly achieved by relying on the parametrized pose like the stick-figure model approach for the stuff members.

In an example, not shown in detail, the output interface 16 is configured to present the radiation information as visualization on surface parts within the medical room.

For example the visualization is provided as a projection on the floor.

In an example, the radiation information is provided restricted to a respective user. In another example, the radiation information is provided to other users as well.

In an example, the radiation information is provided within an augmented reality context. In another example, the radiation information is provided as an ambient light.

In an option, different sensors are provided for tracking the X-ray imaging system.

The prediction of the X-ray radiation can also be referred to as X-ray simulation.

The medical room can also be referred to as X-ray imaging room.

The current scene can also be referred to as current situation.

In an example, images of the scenes in the room are provided showing the personnel and equipment. Next, the personnel in the X-ray room is determined. Further, a pose of personnel, which is based on stick figures, is provided. The determination of position of objects in the X-ray room is including the X-ray source and personnel. Furthermore, based on the position of the X-ray source and the objects in the room, an amount of stray radiation is determined as a function of the respective positions in the room. Based on the stick figures and the stray radiation, the amount of radiation received by each body part of the personnel is determined. Then, a warning or advise to move or adjust your pose and/or position is Generated. Then, the amount of scatter radiation that the person might acquire before the actual radiation starts is predicted such that the personnel can adjust their position and pose prior to starting the radiation. Then, an image analysis in the camera images is performed for the exposed portions to determine the presence of radiation protection means, e.g. aprons, lead glasses, thyroid protection etc...

In an option, an AI trained algorithm is provided to recognize such radiation protection in the camera images.

Fig. 2 schematically shows an example of a system 50 for providing radiation information for a current X-ray imaging procedure. The system 50 comprises an example of the device 10 for providing radiation information for a current situation according to one of the preceding examples. Further, at least one camera 52 is provided. The at least one camera 52 provides the image data about the current scene in the medical room. In Fig. 2, three cameras 52 are shown as an example. In other options, less or more cameras are provided.

A display device 54 is shown which can be a projector for providing the radiation information to the user displayed in situ on surfaces. The display device 54 can also be a monitor.

In an option, multiple cameras are provided to provide images about the current scene in the medical room from different viewing perspectives.

The system 50 can be integrated in an X-ray imaging arrangement. The system 50 can also be installed with existing X-ray imaging arrangements for upgrade purposes.

Fig. 3 shows an example of an X-ray imaging arrangement 100 with radiation information prediction. The arrangement 100 comprises an example of the system 50 for providing radiation information for a current X-ray imaging procedure according to the example above. Further, an X-ray imaging system 102 is provided. The X-ray imaging system 102 is configured to perform the planned X-ray imaging procedure. The X-ray imaging system 102 provides the imaging parameters of the planned X-ray imaging procedure to the device for providing radiation information for the current situation.

The X-ray imaging system 102 is shown as a mobile X-ray imaging device with a mobile platform 104, or mobile base, to which a C-arm 106 is movably mounted. An X-ray source 108 and an X-ray detector 110 are mounted to opposite ends of the C-arm 106. This allows X-ray imaging of a subject from different viewing angles.

The X-ray imaging system 102 is data-connected to the device 10 for providing radiation information for a current situation, as indicated with data-line 112.

In addition, also other equipment is illustrated, such as a mobile stand 114 for a movable light 116. A display arrangement 118 is also shown mounted to the mobile stand 114. Of course, further monitors can also be arranged in different manner, like suspended from the walls and the ceiling.

A subject support 120 is shown with a subject 122. A user interface 124 is shown in the vicinity to the subject support 120.

As an example, a first user 126 and a second user 128 are shown in an active position during a medical activity, e.g. during an intervention or examination procedure.

For a better understanding of the concept to provide radiation information for a current situation, a first simplified line-model 130 is shown overlaid as an example for determining a parametrized pose of the first user 126. As an option, the first simplified line-model 130 is provided as a stick-figure model, in which the anatomy is reduced to lines connected in a pivoting manner. In a similar manner, a second simplified line-model 132 is shown overlaid as an example for determining a parametrized pose of the second user 128.

In another option, the X-ray imaging system 102 is a stationary X-ray imaging system. The X-ray imaging system can be mounted to the floor, the walls or the ceiling.

In further options, other supports and moving concepts are provided for the X-ray source and the X-ray detector. For example, the X-ray source and the X-ray detector are mounted to robot arms.

In. Fig. 3, three cameras 52 are shown above the scene. In other options, the cameras 52 are arranged within the scene, e.g. attached to the equipment or surrounding wall structures.

In an option, the X-ray imaging system 102 is configured to provide information about a spatial arrangement of the X-ray imaging device within the medical room to the device for providing radiation information for the current situation.

In an example, an X-ray room is equipped with one or a plurality of cameras capable of capturing scenes in the room with pose and position estimation of personnel in the room based on stick figures. The cameras are also capable of determining the objects and position in the room, e.g. in particular the X-ray source, X-ray scatter objects, protection shields/equipment and other equipment. It is determined which portions of the model, e.g. segments of the stick figure, are exposed to scatter radiation above a threshold.

As an option, the radiation relates to the to be exposed radiation. In another option, the radiation relates to real time exposure. In another option, the radiation relates to cumulative radiation.

Further, a warning or advise may be generated to move or adjust the pose and/or position of the respective staff member. As an option, an image analysis is performed in the camera images for the exposed portions to determine the presence of radiation protection means such as aprons, lead glasses, thyroid protection and so forth.

In an example, a spatial distribution of the hazardousness of radiation doses is estimated for individuals evolving in a medical operating room defining a three-dimensional environment surrounding the source of radiation. A three-dimensional model of the environment is obtained and a simulation of radiation doses attributable to ionizing radiation emitted from the source and scattered by the environment is computed in the model. The three-dimensional model comprises models of individuals when the individuals are present in the environment and the image is a three-dimensional image generated for at least a portion of the model including said models of individuals. The human body of the individuals are represented by simplified stick figures that are easy to track over time. Further, determining the persons by a set of lines reduces possible errors, e.g. when multiple persons partially are occluding each other. Then, an image indicating the spatial distribution of the hazardousness for an individual of the radiation doses is generated and displayed.

In an option, a number of data inputs in the interventional room is provided. The main data inputs are for example, multiple optical sensors, such as of the shelf cameras. In an option, cameras with depth sensing are provided. The optical sensors provide images from different fixed angles in the room.

In another option, cameras with depth sensing are provided.

In an option, infrared or hyperspectral images are provided.

In a further option, angio system parameters are provided, e.g. 3D positions of the different components of the angio system (C-arm, table) as well as the X-ray acquisition parameters.

A scatter radiation diffusion model definition is provided. e.g. a model definition which describes the distribution of scatter radiation emitted by the angio system in the space. This model definition is a pre-calculated simulation of how scatter radiation would spread based on the X-ray source parameters and X-ray emission, the position of table and basic parameters of the patient.

Additional input could be provided in form of data relating to fixed elements in the space, such as shields attached to the table, and other configurations such as pre-calculated camera calibration models.

As an option, the camera calibration parameters are pre-calculated using algorithms based on pre-recorded videos in the interventional room using standard checker boards and/or well visible 3D objects, such as transparent 3D cubes.

In an example, the protection shield(s) orientation and people body part positions in the 3D space are calculated by 1) extracting the objects coordinates in 2D camera images (using of the shelf pose detecting algorithms such as AlphaPose or OpenPose) and 2) calculating the 3D positions of the objects by triangulating the 2D object coordinates from the different camera angles, using as input the camera calibration parameters.

In parallel, the invention calculates the scatter dose distribution. This is calculated by getting the position of the X-ray source (including the currently selected radiation settings), the table position, available basic patient parameters (such as patient weight range) and based on the available scatter radiation definition model calculates a 3D model of how the radiation would scatter in case the X-ray source emits (at this very moment).

Next, the two 3D models are combined in one view in order to calculate the radiation safe area (which is behind the protection shield(s)) taking into account the various body parts position of the personnel. The 3D orientation of the shield(s) in space will determine the shadow in the 3D scatter radiation model, which is considered a safe zone. Additionally, the combined model calculates the positions of the people relative to this safe zone.

The visualization and the user feedback are also provided. The combined 3D model is suitable for different visualizations and user feedback mechanisms. These can range from an interactive 3D visualization of the room and the objects in it to a simple 2D floor map with the position of the people relative to the safe zone or just an audio/visual signal whenever staff member is outside the safe zone.

In another option, a part of the timeout/preparation phase of the procedure is provided, helping staff determine best safe positions of people and objects in the room.

In an option, the 3D pose algorithm also detects what body parts are covered by lead aprons. For instance, the algorithm may detect when personnel is not wearing lead glasses or is using the lead apron to cover their neck. These observations can then be used to adopt the radiation hazard profile by incorporating this in the calculation.

In a further embodiment, the system tracks over time events that resulted in radiation exposure to certain body parts of the personnel. For these events, the body posture can be stored such that the personnel can look back what was the cause of the radiation exposure to be able to learn how to do things different in the future to minimize these exposures.

In a further embodiment, usages of the lead shield can be tracked over time during the procedure in combination of the 3D pose of the personnel. Furthermore, the deviation from the optimal position of the lead shield can be determined, e.g. calculated with AI techniques. From this the personnel can learn how to improve the lead shield usage in the future.

In an option, a calibration of the cameras is provided for each new imaging,

In an option, the calibration of the cameras is provided only upon installation.

In an example, the calibration of the cameras is provided in intervals.

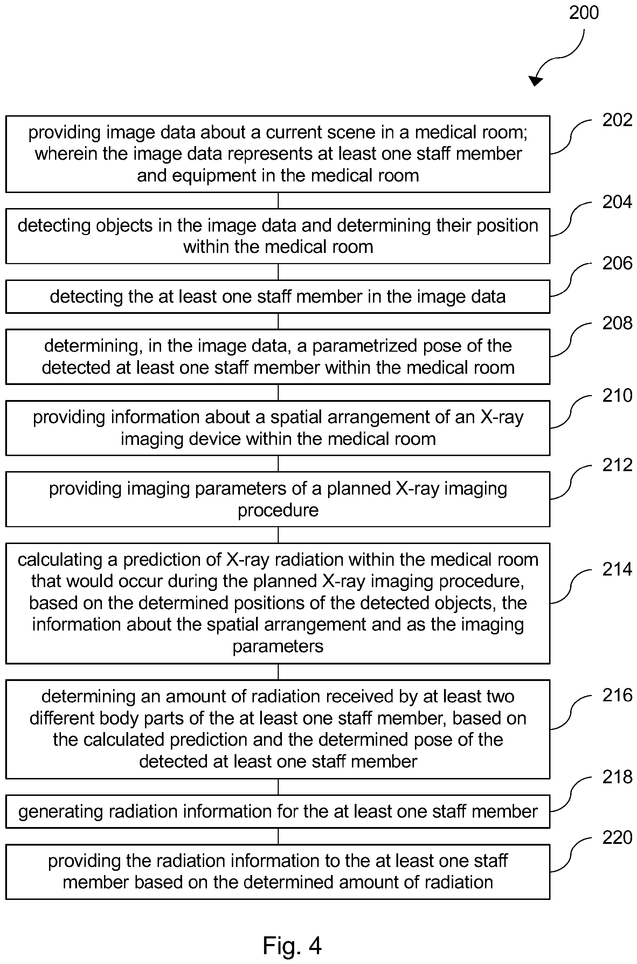

Fig. 4 shows basic steps of an example of a method 200 for providing radiation information for a current situation. The method 200 comprises the following steps: In a first step 202, image data about a current scene in a medical room is provided. The image data represents at least one staff member and equipment in the medical room. In a second step 204, objects in the image data are detected and their position within the medical room is determined. In a third step 206, the at least one staff member is detected in the image data. In a fourth step 208, in the image data, a parametrized pose of the detected at least one staff member within the medical room is determined. In a fifth step 210, information about a spatial arrangement of an X-ray imaging device within the medical room is provided. In a sixth step 212, imaging parameters of a planned X-ray imaging procedure are provided. In a seventh step **214,** a prediction of X-ray radiation within the medical room is calculated that would occur during the planned X-ray imaging procedure, based on the determined positions of the detected objects, the information about the spatial arrangement and as the imaging parameters. In an eighth step 216, an amount of radiation received by at least two different body parts of the at least one staff member is determined, based on the calculated prediction and the determined pose of the detected at least one staff member. In a nineth step 218, radiation information for the at least one staff member is generated. In a tenth step 220, the radiation information is provided to the at least one staff member based on the determined amount of radiation.

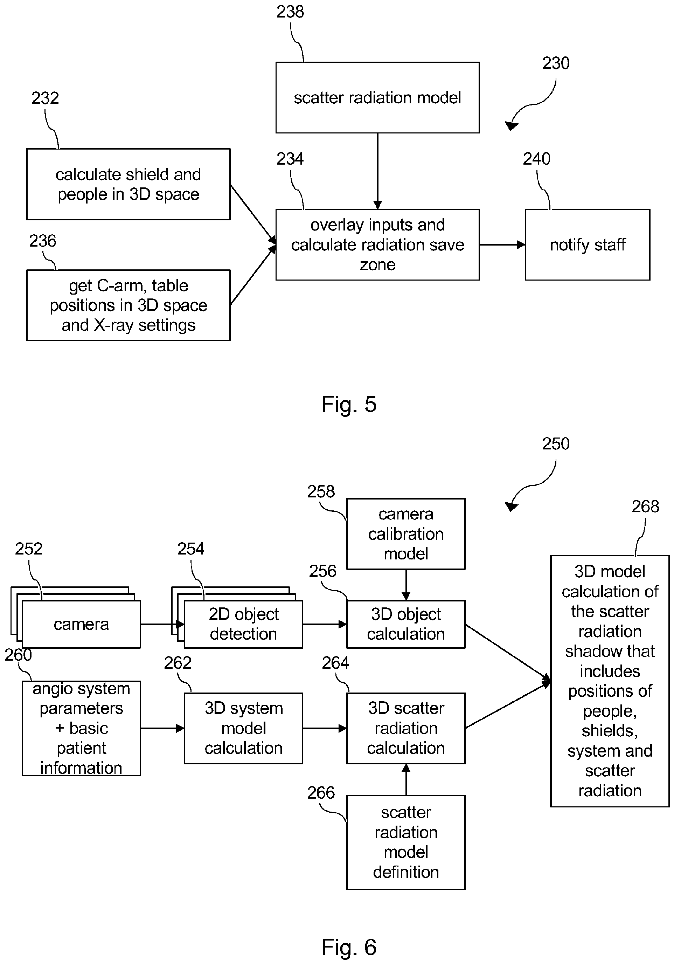

Fig. 5 shows an example of a working scheme 230. In a first frame 232 indicates that shield(s) and people are calculated for a 3D space. This is provided to a next frame 234. In addition, a second frame 236 indicates that C-arm and table positions for a 3D space are received. This is also provided to the next frame 234. Furthermore, a scatter radiation model 238 is provided to the next frame 234, in which the inputs are overlaid, and a radiation save zone is calculated. This is then provided to notify the staff, indicated by a result frame 240.

Fig. 6 shows an example of another working scheme 250. One or more cameras 252 are provided for 2D object detection 254 in the image data. Depending on the number of images, several 2D object detections are provided. The result is provided to a 3D object calculation 256, which also receives a camera calibration model 258. In addition to the camera images 252, also angio system parameters and basic patient information is provided, as indicated with frame 260. This is used for a 3D system model calculation 262. This is provided to a 3D scatter radiation calculation 264, which also receives a scatter radiation model definition 266. The results of the 3D object calculation 256 and the 3D scatter radiation calculation 264 are provided to 3D model calculation of the scatter radiation shadow that includes positions of people, shields, system and scatter radiation, as indicated with frame 268.

In an example, a computer program is provided comprising instructions which, when the program is executed by a computer, cause the computer to carry out the method of the example above.

In an example, not shown in detail, a computer program or program element for controlling an apparatus according to one of the examples above is provided, which program or program element, when being executed by a processing unit, is adapted to perform the method steps of one of the method examples above.

In an example, a computer readable medium having stored the computer program of the method example above is provided.

The sensor and data inputs, the data processing and calculation, plus the Visualization and the user feedback can be provided in a (near) real-time mode.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for providing radiation information for a current situation, comprising:
- a data input (12);
- a data processor (14); and
- an output interface (16);
wherein the data input is configured: to provide image data (18) about a current scene in a medical room, the image data representing at least one staff member and equipment in the medical room; to provide information (20) about a spatial arrangement of an X-ray imaging device within the medical room; and to provide imaging parameters (22) of a planned X-ray imaging procedure;
wherein the data processor is configured: to detect objects in the image data and to determine their position within the medical room; to detect the at least one staff member in the image data and to determine, in the image data, a parametrized pose of the detected at least one staff member within the medical room; to calculate a prediction of X-ray radiation within the medical room that would occur during the planned X-ray imaging procedure, based on: the determined positions of the detected objects and the information about the spatial arrangement and imaging parameters; to determine an amount of radiation received by at least two different body parts of the at least one staff member, based on the calculated prediction and the determined pose of the detected at least one staff member; and to generate radiation information for the at least one staff member; and
wherein the output interface is configured to provide the radiation information to the at least one staff member based on the determined amount of radiation.

2. Device according claim 1, wherein the parametrized pose of the detected at least one staff member within the medical room is based on a stick-figure model.

3. Device according to claim 1 or 2, wherein the radiation information comprises a visualization of the radiation distribution in the spatial context in relation to the at least one staff member and the staff member's at least two body parts.

4. Device according to claim 1, 2 or 3, wherein the data processor is configured to determine the amount of radiation as a prediction before an actual imaging takes place.

5. Device according to one of the preceding claims, wherein, for providing the information about the spatial arrangement of the X-ray imaging device within the medical room, the data processor is configured to detect the X-ray imaging device in the image data and to determine its spatial arrangement.

6. Device according to one of the preceding claims, wherein the data input is configured to provide information about radiation protective measures within the medical room; and
wherein the data processor is configured to determine the amount of radiation also based on the information about the radiation protective measures; and
wherein, preferably, for providing the information about the radiation protective measures, the data processor is configured to analyze image data for identifying radiation protective measures and for determining their spatial arrangement.

7. Device according to one of the preceding claims, wherein the output interface is configured to provide a temporal presentation of the determined radiation in order to show where and when radiation is occurring.

8. Device according to one of the preceding claims, wherein the image data is provided as data from an optical camera.

9. Device according to one of the preceding claims, wherein the information about the spatial arrangement of the X-ray imaging device within the medical room also comprises data of a subject support; and
wherein, preferably, the image data also comprises image data of a subject.

10. Device according to one of the preceding claims, wherein the data processor is configured to update the determined amount of radiation constantly reflecting changes in the current scene.

11. Device according to one of the preceding claims, wherein the output interface is configured to present the radiation information as visualization on surface parts within the medical room.

12. A system (50) for providing radiation information for a current X-ray imaging procedure, the system comprising:
- a device (10) for providing radiation information for a current situation according to one of the preceding claims; and
- at least one camera (52);
wherein the at least one camera provides the image data about the current scene in the medical room.

13. An X-ray imaging arrangement (100) with radiation information prediction, the arrangement comprising:
- a system (50) for providing radiation information for a current X-ray imaging procedure according to claim 12; and
- an X-ray imaging system (102);
wherein the X-ray imaging system is configured to perform the planned X-ray imaging procedure; and
wherein the X-ray imaging system provides the imaging parameters of the planned X-ray imaging procedure to the device for providing radiation information for the current situation.

14. A method (200) for providing radiation information for a current situation, the method comprising the following steps:
- providing (202) image data about a current scene in a medical room; wherein the image data represents at least one staff member and equipment in the medical room;
- detecting (204) objects in the image data and determining their position within the medical room;
- detecting (206) the at least one staff member in the image data;
- determining (208), in the image data, a parametrized pose of the detected at least one staff member within the medical room;
- providing (210) information about a spatial arrangement of an X-ray imaging device within the medical room;
- providing (212) imaging parameters of a planned X-ray imaging procedure;
- calculating (214) a prediction of X-ray radiation within the medical room that would occur during the planned X-ray imaging procedure, based on the determined positions of the detected objects, the information about the spatial arrangement and as the imaging parameters;
- determining (216) an amount of radiation received by at least two different body parts of the at least one staff member, based on the calculated prediction and the determined pose of the detected at least one staff member;
- generating (218) radiation information for the at least one staff member; and
- providing (220) the radiation information to the at least one staff member based on the determined amount of radiation.

15. Computer program comprising instructions which, when the program is executed by a computer, cause the computer to carry out the method of claim 14.
